Europäisches Patentamt

European Patent Office

Office européen des brevets

(11) Publication number: **0 063 604**
A1

# EUROPEAN PATENT APPLICATION

published in accordance with Art. 158(3) EPC

(21) Application number: 81902607.1

(22) Date of filing: **25.09.81**

Data of the international application taken as a basis:

(86) International application number: **PCT/JP 81/00254**

(87) International publication number: **WO 82/01129 (15.04.82 82/10)**

(51) Int. Cl.³: **A 61 K 9/70, A 61 L 15/03**

(30) Priority: **26.09.80 JP 133947/80**

(43) Date of publication of application: **03.11.82 Bulletin 82/44**

(84) Designated Contracting States: **CH DE FR GB LI NL**

(71) Applicant: **NIPPON SODA CO., LTD., 2-1, Ohte-machi 2-chome, Chiyoda-ku, Tokyo, 100 (JP)**

(72) Inventor: **KIZAWA, Hidenori, 33-15, Kamisoshigaya Setagaya-ku, Tokyo 157 (JP)**
Inventor: **FUJIYAMA, Norimasa, 7-25 Higashigaoka 2-chome Meguro-ku, Tokyo 152 (JP)**
Inventor: **KOBAYASHI, Jitsuo, 7-6 Midorigahama Chigasaki-shi, Kanagawa 253 (JP)**
Inventor: **ITO, Akinori, 4846-1 Ohnuki Jhoetsu-shi, Niigata 943 (JP)**

(74) Representative: **van der Beek, George Frans et al, Nederlandsch Octrooibureau Johan de Wittlaan 15 P.O. Box 29720, NL-2502 LS Den Haag (NL)**

(54) **MUCOUS MEMBRANE-ADHERING FILM PREPARATION AND PROCESS FOR ITS PREPARATION.**

(57) A mucous membrane-adhering film preparation is applied to mucous membrane or inflamed portion thereof to obtain general or local curing effects. This film preparation is prepared by forming a drug-containing, water-soluble high polymer film and making one side thereof difficulty water-soluble.

-1-

SPECIFICATION

Title of the Invention

Mucous membrane-adhering film preparation and process for its preparation

Field of Technique

The present invention relates to a preparation of applying on a mucous membrane and more particularly, it relates to a mucous membrane-adhering film preparation which is plastered and adherred on a mucous membrane part or an inflamed portion to obtain general or local curing effects, and a process for its preparation.

Background of Technique

A preparation of applying on mucous membrane is paticularly attracted in the recent years to have expectation of quick curing effects by reason of that a high concentration of a pharmaceutical agent is absorbed by a mucous membrane, and carried directly to a whole body by a great circulation system, and to have a lesser loss of the pharmaceutically effective compornent in which the said loss occurs by a decomposition and chemical or microorganism reactions of the pharmaceutically effective compornent with the PH condition, a digestive juice and microorganism in a gastric and intestinal organs, such as general oral drugs of which the pharmaceutical effective compornent is obsorbed by a gastric wall and intestinal organs and carried to a whole body possing through the portal veins and heptic organ.

A troche, a buccal tablet and a tonque tablet as a preparation of applying on mucous membrane in the oral cavity, a suppository and a vagina suppository for applying to rectum and vagina, and an ointment and an adhering tablet as an externally employable preparation of applying directly to the inflamed portion are well known, as a preparation of applying on the mucous membrane.

0063604

However, if the external use preparation is examplified, it is difficult to coat the suffered part alone with the ointment, particularly, in a buccal cavity, a stagnating time of pharmaceutical component on a ulcer part is short in the consequence of saliva and a move of tongue and cheeks, and a pharmaceutical effect is not sufficient in comparison with its dosage amount and this is a drawback. Further, the adhering tablet applied for the larger ulcer stimulates and increases rather a contact pain and furthermore in the event of applying it on the several ulcers, plural pieces of adhering tablets must be plastered on those ulcers and thereby an extraordinary impression of extraneous material is felt on the plastered parts and this is a drawback.

As preparations having improved those drawback, a preparation of an adhering type on a mucous membrane in the oral cavity or nasal cavity was proposed in the 99th annual meeting of Japan Pharmaceutical Society (August 28, 1979) and disclosed in Public Bulletin of Japanese Open Patent Publication No. 100714/1981 in which the base agent of the ointment is eccentrically pleaced in a mucous membrane-adhering coating layer such as cellulose ether.

The said preparation has an improved soft flexibility compared with a conventional adhering tablet, but maked size of the preparation is limitedly defined so that other drawbacks are settled.

The inventors carried out the eager researches as to the preparation of applying to the mucous membrane and as its results they discovered the film preparation that the one surface of the water-soluble high polymer film containing a pharmaceutical agent is treated as the difficulty water-soluble surface and thus, they accomplished the present invention. An object for the present invention is to provide the mucous membrane-adhering film preparation and another object is to provide a process for producing the said film preparation.

Disclosure of the Invention

The present invention comprises

(1)   the mucous membrane-adhering film preparation in which the one surface of the water-soluble high polymer film containing pharmaceutical agents is treated as difficulty water-soluble surface, and

(2)   the process for producing the mucous membrane-adhering film preparation in which a solution containing the pharmaceutical agent and the water-soluble high polymer and another solution containing the agent to be difficulty water-soluble and the water-soluble high polymer are separately prepared and then, the one solution, out of above two solution is coated on a base plate having a favourable releasing nature, and by removing its solvent, the film is produced on the said base plate and then, the other solution is coated on the said former film, and by removing its solvent, the objective film having one difficulty water-soluble surface is produced.

The pharmaceutical agent for the present invention comprises medical agents or animal agents which apply to a mucous membrane part or a inflamed portion on a mucous membrane and cures therapeutically or prevents the diseases in whole body or local portion.  Further, it involves pharmaceutical agents which can be dissolved or dispersed in water or organic solvents at room temperature and it is desirably chosen from pharmaceutical agents which discharges gradually an effective component whereby a great deal of therapeutical effect can be expected.

For example, it comprises predonisone, predonisolone, predonisolone acetate, hydrocortisone, triamcinolone, dexamethasone, and betamethasone as anti-inflammatory steroids and aspirin, aminopyrin, acetoaminophen, ibufenac, ibuprofen, indomethasine, colchicine, sulpyrine, mephenamic acid, phenacetin, phenylbutazon, fulfenamic acid and probenecid as anti-inflammatory anodynes, ($\alpha$)-chymotrysin as anti-inflammatory enzymes.  Further, it comprises anti-histamine agents such as diphenhydramine-hydrochloride, and chlorophenylamine maleate, oral sterilizing agents such chlorohexydine-hydrochloride, cetylpyridinium-chloride, hexylresorcin and nitrofurazone, antibiotic materials such as penicillin or its derivative, cephaphalosporin derivative, erythromycine, tetracycline hydrochloride, furadiomycin, and leucomycin, chemically therapeutical agents such as sulfamethyzole and nalidixic, cardiac strengthening agents such as digitalis and digoxin, blood vein dilating agents such as nitroglycerine and papaverine-hydrochloride, local narcotic agents such as lidocaine and procaine-hydrochloride, cough curing agents such as codeine phosphate and bisorlvon, digesting organ curing agents such as azulene, phenovalin, pepsin and vitamin U, enzymes such as lysozyme-chloride or trypsin and antidiabetic agents such as insulin. Furthermore, it comprises other kind agents such as depressing blood pressure agents, tranquilizers, styptic agents sexual hormones and agents of curing virulent carcinoma or ulcer.

The water-soluble high polymer in the present invention is used for a film base material producing the film preparation and it comprises a natural or syntheticwater-soluble high polymer having a superior film moldability and does not give a pharmaceutically bad influence on human body or animal body, but it is able to produce the soft flexible film.

For example, it involves water-soluble cellulose derivatives such as hydroxypropyl cellulose (hereinafter it is abbreviated as "HPC"), methyl cellulose, hydroxypropyl alkylcellulose, carboxymethyl cellulose or its salt. Further, it comprises an acrylic acid copolymer or its sodium, potassium or ammonium salt which is obtained by a copolymerization with poly-acrylic acid or its sodium, potassium or ammonium salt as a main component and methacrylic acid, styrene or vinyl type of ether (i.e. methyl vinylether or the like) as a comonomer, poly vinyl alcohol, poly vinyl pyrrolidone, polyalkylene glycol, hydroxypropyl starch, alginic acid or its salt, polysacoharides or those derivatives such as tragacanth rubber, gelatin, collagen or denatured gelatin and collagen treated with succinic acid or anhydrous phthalic acid.

As the said water-soluble high polymer, one kind or two kinds or more of above examplifiedhigh polymer(s) may be used, but cellulose derivatives having superior film moldability and producing the soft flexible film are desirably used, and further the "HPC" out of numerous cellulose drivatives, discharging gradually the effective component is most desirably used.

A process for feeding the difficulty water-solubility on the single surface of the water-soluble high polymer film in the present invention is to provide the difficulty water-soluble layer on the said single surface of the water-soluble high polymer film, in which the water-soluble high polymer film containing agents to be difficulty water-soluble is laminated on the single surface of the water-soluble high polymer film, or the single surface of the water-soluble high polymer film is partially crosslinked with an irradiation step of radiation ray or infrared ray, and thereby the single surface the said water-soluble high polymer film is treated with an difficulty water-soluble.

As the process for feeding the single surface of the water-soluble high polymer film with the difficulty water-soluble layer, the former process mentioned above can be easily carried out. The former process is not feared of denaturing the pharmaceutical agent with the difficulty water-solubilizing treatment, and consequently it is desirably used.

As agents to be difficulty water-soluble employable for the difficulty water-soluble layer to the water-soluble high polymer film, a shellac (product of dispensary Bureau), higher fatty acids involving stearic acid and palmitic acid or the like and difficulty water-soluble cellulose derivatives involving ethyl cellulose, cellulose acetate and butyl cellulose may be used.

The mucous membrane-adhering film preparation in the present invention is a filmy pharmaceutical preparation in which the single surface of the water-soluble high polymer film containing the pharmaceutical agent is treated with the difficulty water-soluble layer by above mentioned process.

The film preparation is a pharmaceutical preparation applying on the mucous membrane, in which the said film preparation is cut as a desirable size in accordance with the affected diseased part and a dosage amount of pharmaceutical component and it is used by plastering, adhering on mucous membrane in oral cavity, nasal cavity, rectum and vagina, and thereby a therapeutical effect is obtained on a whole body and/or local parts.

If desirable, a plasticizer, a bulking agent, a tast modifying agent, an odour modifying agent and a pigment, as additives may be added in the mucous membrane-adhering film preparation of the present invention.

The plasticizer employed for the purpose of feeding appropriately the soft flexibility to the film preparation is examplified as polyethylene glycol (macrogol), propyleneglycol, glycerin, copolymer with ethylene oxide and propylene oxide, spun type of fatty acid-lauric ether or an adduct compound in which ethylene oxide or propylene oxide is added in a containing active hydrogen atom such as cane sugar, sorbitol, glycerin or pentaerythritol. The polythylene glycol (macrogol) is desirably used in the event of employing the "HPC" as the water-soluble high polymer.

As the bulking agent, an exeipient agent which is added in a conventional tablet is used and it involves avicel, mannitol, lactic acid, sorbitol, dextrin, starch, anhydrous calciumphosphate or amylose.

As a taste improving agent, organic acid compounds of feeding sour taste such as citric acid, tartaric acid, fumaric acid or the like, and sweetening agents such as saccharin, glycyrrhizin or the like, and a menthol are used.

As an odour modifying agent, a natural or synthetic perfume agent is used and as a pigment, an edible lake pigment being added in a conventional tablet is used.

The mucous membrane-adhering film preparation by the present invention is produced with a subsequent process:

A solution containing one kind or two kinds or more of the said pharmaceutical agent(s) and one kind or two kinds or more of the said water-soluble high polymer(s) is blended with one kind or two kinds or more of additive(s) and thereby the solution (A) is prepared. A solution containing one kind or two kinds or more of the said agent(s) to be difficulty water-soluble and one kind or two kinds or more of the said water-soluble high polymer(s) is if desirable blended with one kind or two kinds or more of the said additive(s) and thereby, the solution (B) is prepared.

The solvents for the said solution (A) and the said solution (B) are changeable depending on the kinds of the employable water-soluble high polymer(s) pharmaceutical agent(s), agent(s) to be difficulty water-soluble and additive(s) and these concentrations. Water, methanol, ethanol, isopropanol, acetone, methylene chloride and cellusolve or the like may be individually used and further two kinds or more of these solvent may be used by taking in consideration the volatile nature of the solvent as the said solvent. If a control for residual solvent in the pharmaceutical agent is taken in consideration, water and/or ethanol are desirably used.

In the event of preparing the said solution (B) by using for example, the "HPC" as the water-soluble high polymer an employing amount of the agent to be difficulty water-soluble is chosen as weight ratio of HPC/shellac in a range of 9/1 to 1/9 and as weight ratio of HPC/higher fatty acid in a range of 9/1 to 7/3 and the said weight ratio may be optionally chosen by considering a desirable degree of difficulty water-solubility.

One solution, out of the solution (A) and the solution (B) prepared above is coated on a base plate having favourable releasing nature, for example, teflon plate or glass plate and then, by removing the solvent, the film is produced on it, and further another solution, out of the said solutions (A and B) is coated on the former film and thereby, the film preparation having the difficulty water-soluble surface can be produced. The coating procedure of the solution (A) or the solution (B) on the base plate or the prior coated film can be carried out by a brush coating step or an atomizing spraying step. But, by means of setting a frame mould around the base plate and pouring the solution on the base plate, a thickness of the film can be easily adjusted and this operation is simple and desirable.

A removal of the solvent posterior to the coating step of the solvent is carried out by heating slightly the coated film under an atomospheric pressure or a reduced pressure.

The film preparation having an optional thickness can be produced withabove mentioned process and the film preparation having a total thickness of 1 mm or less does not give an extraneous material contacting feeding when it is plastered and adherred on the effected mucous membrane. So, it is desirable. The difficulty water-soluble layer in the said film preparation has usually a thickness of 0.01 to 0.9 mm.

In the present invention, a layer which is free from the pharmaceutical agent and the agent to be difficulty water-soluble (hereinafter, this is called as and "intermediate layer") may be intermediately inserted between a layer containing the pharmaceutical agent (hereinafter, this is called as "pharmaceutical agent layer") and another layer containing the to be difficulty water-soluble (hereinafter, this is called as "difficulty water-soluble layer").

Brief Illustration to the Drowing:

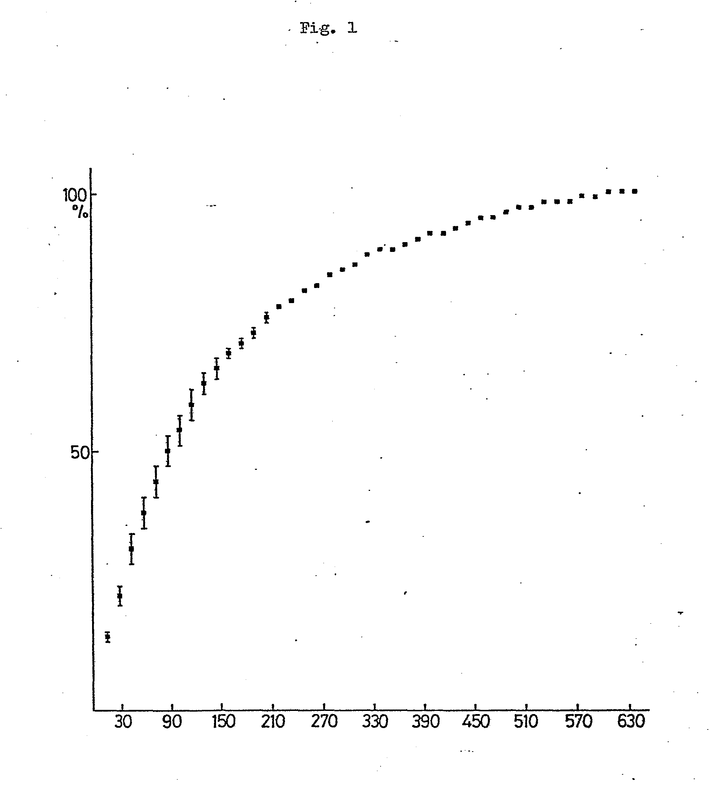

Fig. 1 shows an elution testing result of the pharmaceutical agent from the film preparation which is obtained in subsequent Example [1]-2 of the present invention. In its diagram, the ordinate denotes the elution ratio of the pharmaceutical agent (%) and the abscissa denotes an elapsed time (minute).

The Best Mode for Performance of the Present Invention:

Examples and Experimental Examples in the best mode for carrying out the present invention are set forth below. But, the present invention is not difined limitedly by the following Examples and Experimental Examples.

Example [1]

(i) Preparation for the film base agent

(a) The solution of the film base agent for preparing the pharmaceutical layer:

3.5 g of hydroxypropyl cellulose and 0.5 ml of macrogol-400 (polyethylene glycol) were dissolved in 63 g of ethyl alcohol and 10 ml of distilled water containing 100 ml of dissolved predonisolone was added in the resulting solution.

(b) The solution of the film base agent for preparing the intermediate layer:

3.5 g of hydroxypropyl cellulose and 0.5 ml of macrogol-400 (polyethylene glycol) were dissolved in 63 g of ethyl alcohol.

(c) The film base agent solution for preparing the difficulty water-soluble layer:

0.5 g of hydroxypropyl cellulose, 0.25 ml of macrogol (polyethylene glycol) and 0.25 g of shellac were dissolved in 6 g of ethyl alcohol.

(ii) The film moulding process:

The film base agent solution for producing the pharmaceutical agent layer (a) was firstly poured on the film moulding frame (i.e. teflon plate) having 10 cm$^2$ of surface area, being placed a horizontal position. After its drying step, the film base agent solution for preparing the intermediate layer (b) was poured on the film containing the pharmaceutical agent and then, after it was dried, the film base agent solution for preparing the difficulty water-soluble layer (c) was injected on it. After its semi-drying step, the laminated film was stripped from the said film moulding frame. The stripped film was cured at 50°C of temperature during a half day. The produced film consists of three layers and it has a transparency and a soft flexibility.

Further, by almost same operation, three laminated layer films having different combination of compositions were obtained as set forth in Table 1.

Example [2]

In the stead of the said predonisolone as the film base agent of the pharmaceutical layer shown as Example [1], the allantoin (i.e. 3-ureido hydantoin) was employed. The two laminated films having different combination of the compositions were obtained from the film base agent solution containing the allantoin and the film base agent solution producing the difficulty water-soluble layer as as set forth in Table 2.

Experimental Example [1]

By using the film preparation in Example [1]-2, an elution test for the pharmaceutical component "Predonisolone" was carried out. The testing result is set forth in Fig. 1.

The Elution Testing Apparatus:

Product "VSP (NF)" of Toyama Industry Co., Ltd. Standard Grade: NTR-55. Aq.

Dest. 1,000 ml, 37°C and basket having 100 rpm of rotation velocity.

- 11 -

The Automatic Sampling Apparatus:

Product "TAS-30 TC II" of Toyama Industry Co., Ltd.  Sampling amount:  10 ml
and Interval:. 15 minutes.


Experimental Example: [2].  _

The film preparation obtained in the Example [1] was applied to the patient
suffered from "After" type of oral inflamed disease.

As one clinical example, the film preparation is cut as a size slightly
larger than the affected area, so as the cut piece may contain 2 to 16 mg of
predonisolone and the said cut piece was adherred on the affected part.

As the results, the film preparation showed a favourable adhesiveness and
it was not stripped down in the course of its therapeutical disposal and as
to all applying Examples, a quick curing effect was recognized.

Further, in the event of using the film preparation by the present invention, the affected part is protected with a soft film and a physical stimulus
by teeth or food can be avoided.  Consequently, the pains which is a largest
stress of this disease was remarkably mitigated.  As the comparison in this
respect, the predonisolone tablet having 1.2 mm of thickness was adherred on the
affected part and in this occasion, a residual pains was recognized.

Table 1

| Example No. | [1]-1 | | | [1]-2 | | | [1]-3 | | | [1]-4 | | | [1]-5 | | |
|---|---|---|---|---|---|---|---|---|---|---|---|---|---|---|---|
| | Pharmaceutical agent layer | Intermediate layer | Difficulty water-soluble layer | Pharmaceutical agent layer | Intermediate layer | Difficulty water-soluble layer | Pharmaceutical agent layer | Intermediate layer | Difficulty water-soluble layer | Pharmaceutical agent layer | Intermediate layer | Difficulty water-soluble layer | Pharmaceutical agent layer | Intermediate layer | Difficulty water-soluble layer |
| Predoni-solone | 100 mg | – | – | 100 mg | – | – | 100 mg | – | – | 100 mg | – | – | 100 mg | – | – |
| Hydroxy-propyl cellulose | 3.5 g | 3.5 g | 0.5 g | 3.5 g | 2.5 g | 0.5 g | 3.5 g | 2.5 g | 0.5 g | 2.0 g | 3.5 g | 0.3 g | 2.0 g | 3.5 g | 0.3 g |
| Hydroxy-propyl methyl-cellulose | – | – | – | – | – | – | – | – | – | 1.5 g | – | 0.2 g | – | – | 0.2 g |
| Methyl cellulose | – | – | – | – | – | – | – | – | – | – | – | – | 1.5 g | – | – |
| Macrogol 400 | 0.5 ml | 0.5 ml | 0.25 ml | 0.5 ml | 0.5 ml | 0.25 ml | 0.5 ml | 0.5 ml | 0.25 ml | 0.6 ml | 0.5 ml | 0.3 ml | 0.6 ml | 0.5 ml | 0.3 ml |
| Shellac | – | – | 0.25 g | – | – | 0.25 g | – | – | 0.5 g | – | – | 0.25 g | – | – | 0.25 g |
| The used solvent Ethyl alcohol : water | 10 : 0 | | | 10 : 0 | | | 10 : 0 | | | 8.0 : 2.0 | | | 7.0 : 3.0 | | |

12

0063604

Table 2

| | [2]-1 | | [2]-2 | | [2]-3 | |
|---|---|---|---|---|---|---|
| | Pharmaceutical agent layer | Difficulty water-soluble layer | Pharmaceutical agent layer | Difficulty water-soluble layer | Pharmaceutical agent layer | Difficulty water-soluble layer |
| Allantion | 2.0 g | | 2.0 g | | 2.0 g | |
| Hydroxypropyl cellulose | 3.5 g | 0.75 g | 3.5 g | 0.5 g | 3.75 g | 0.25 g |
| Macrogol 400 | 1.0 ml | 0.25 ml | 1.0 ml | 0.25 ml | 1.0 ml | 0.25 ml |
| Shellac | - | 0.25 g | - | 0.5 g | - | 0.75 g |
| The used solvent | Ethyl alcohol | | Ethyl alcohol | | Ethyl alcohol | |

13

The possibility for the Industrial Utilization

The mucous membrane-adhering film preparation by the present invention is a complex film preparation which consists of the pharmaceutical layer and the difficulty water-soluble layer or the pharmaceutical layer, the intermediate layer and the difficulty water-soluble layer and consequently, the said pharmaceutical layer is strongly adherred on the affected part by absorbing a moisture content and then, the pharmaceutical layer is wetted and swollen. Subsequently, the pharmaceutical agent is gradually dissolved in the affected part in the course of a long hours and thereby the pharmaceutical agent can be almost homogeneously eluted.

On the other hands, the difficulty water-soluble layer is feeded with the difficultly water-solublity and consequently, the pharmaceutical component of the pharmaceutical layer is difficultly eluted and it can be retained in a buccal cavity or the like in long hours without giving a feeling of contacting different admixture or a feeling of contacting extraneous material.

As mentioned above, the said film preparation indicates a superior effect which is entirely different from that in a conventional technique and pharmaceutical preparation.

The features for the film preparation by the present invention are cited out as the following items.

(1) The said film preparation can be used for therapeutical purposes of local affected portion or whole body suffered from disease.

(2) Because of the gradual discharging effect of pharmaceutical component, the effecting hours is long and the lesser stimulus is given on the affected portion.

(3) The said film preparation can be used for the mucous membrane or the affected portion wetted with a diffused liquid or a secreted liquid.

(4)  The said film preparation can be cut as a definite size and thereby a
     necessary amount of the pharmaceutical agent may be used.

(5)  The said film preparation protects the affected portion from physical
     stiumulus.

(6)  Multifarious kinds of pharmaceutical agents can be used for the said film
     preparation.

(7)  The said film preparation has a adhesiveness.

(8)  The said film preparation has a saft flexibility.

(9)  Even if the film base agent is swallowed through oral dosage, it is not
     absorbed in walls of internal organs.

(10) The said film preparation has a low toxicity and a safety.

     As mentioned above, the present invention is to provide the novel film
preparation for applying the mucous membrane and its producing process and it
has a great deal of influences and significances in a view point of pharmacology
as well as industry.

WHAT IS CLAIMED IS

(1) The mucous membrane-adhering film preparation in which the one surface of water-soluble high polymer film containing pharmaceutical agents is treated with difficulty water-soluble.

(2) According to the Claim 1, the said mucous membrane-adhering film preparation which is applied to a mucous membrane or an inflamed portion on a mucous membrane.

(3) According to the Claims 1 and 2, the said mucous membrane-adhering film preparation which is applied on the mucous membrane in a buccal cavity.

(4) According to the Calims 1 to 3, the said mucous membrane-adhering film preparation which comprises using predonisolone or allantoin as its pharmaceutical agent.

(5) The said mucous membrane-adhering film preparation according the Claims 1 to 4, in which the said pharmaceutical agent is discharged grodually.

(6) A process for producing the mucous membrane-adhering film preparation in which a solution containing a pharmaceutical agent and the water-soluble high polymer and another solution containing the agent to be difficulty water-soluble and the water-soluble high polymer are separately prepared and then, the one solution, out of above two solutions is coated on a base plate having a favourable releasing nature by removing its solvent and the film is produced on the said base plate and then, the other solution is coated on the former film, and by removing its solvent, the objective film having one difficulty water-soluble surface is produced.

(7) The said process according to the Claim 6, in which the same kind or the different kind of cellulose derivative are used as the water-soluble high polymer.

(8) The said process according to the Claims 6 and 7, in which one kind or two kinds or more of hydroxypropyl-cellulose, hydroxypropyl-methyl cellulose and/or methyl cellulose are used as the water-soluble high polymer.

(9) The said process according to the Claim 6, in which one kind or two kinds or more of shellac and/or higher fatty acid group are used as the said agent to be difficulty water-soluble.

(10) The said process according to the Calim 6, in which additives involving a plasticizer, a building agent, a taste improving agent, an odour improving agent and a pigment are added in the solution.

(11) The said process according to the Claim 6, in which water and/or ethanol are used as the said solvent.

(12) The said process according to the Claim 6, in which a Teflon plate is used as the said base plate having favourable releasing nature.

(13) The said process according to the Claim 6, in which a coating step of the solution on the base plate or the said former film is carried out by pouring the solution inside a frame fitted around a peripheral circumference of the said base plate.

(14) The said process according to the Claim 6, in which removing step of the solvent from the coated film is carried out with a slightly heating procedure under an atmospheric pressure or a reduced pressure.

1

Fig. 1

# INTERNATIONAL SEARCH REPORT

International Application No  PCT/JP81/00254

| I. CLASSIFICATION OF SUBJECT MATTER (if several classification symbols apply, indicate all) 3 |
|---|
| According to International Patent Classification (IPC) or to both National Classification and IPC |
| Int. Cl.³  A61K 9/70, A61L 15/03 |

## II. FIELDS SEARCHED

| Minimum Documentation Searched 4 | |
|---|---|
| Classification System | | Classification Symbols |
| I P C | A61K 9/20, A61K 9/22, A61K 9/70, A61F 13/02, A61L 15/03, A61L 15/04, A61L 15/06 |

| Documentation Searched other than Minimum Documentation to the Extent that such Documents are Included in the Fields Searched 5 |
|---|
| |

## III. DOCUMENTS CONSIDERED TO BE RELEVANT 14

| Category * | Citation of Document, 16 with indication, where appropriate, of the relevant passages 17 | Relevant to Claim No. 16 |
|---|---|---|
| A | JP,A, 50-105814 (Merck & Co., Inc.) 20, August, 1975 (20.08.75) | 1 - 14 |
| A | JP,A, 55-62012 (Teijin Limited), 10, May, 1980 (10.05.80) | 1 - 14 |
| P | JP,A, 56-20514 (Nitto Electric Industrial Co., Ltd.), 26, February, 1981 (26.02.81) | 1 - 14 |
| P | JP,A, 56-36413 (C.H. Behringer Sohn) 9, April, 1981  (9.04.81) | 1 - 14 |

"A" document defining the general state of the art which is not considered to be of particular relevance

"P" document published prior to the international filing date but later than the priority date claimed

* Special categories of cited documents: 15

"A" document defining the general state of the art

"E" earlier document but published on or after the international filing date

"L" document cited for special reason other than those referred to in the other categories

"O" document referring to an oral disclosure, use, exhibition or other means

"P" document published prior to the international filing date but on or after the priority date claimed

"T" later document published on or after the international filing date or priority date and not in conflict with the application, but cited to understand the principle or theory underlying the invention

"X" document of particular relevance

## IV. CERTIFICATION

| Date of the Actual Completion of the International Search 2 | Date of Mailing of this International Search Report 2 |
|---|---|
| December 20, 1981 (20.12.81) | January 18, 1982 (18.01.82) |
| International Searching Authority 1 | Signature of Authorized Officer 20 |
| Japanese Patent Office | |

Form PCT/ISA/210 (second sheet) (October 1977)